# EUROPEAN PATENT APPLICATION

(11) **EP 2 502 588 A1**
(43) Date of publication of application: **26.09.2012**
(21) Application number: 12160802.0
(22) Date of filing: 22.03.2012
(51) Int. Cl.: A61B 17/34, A61B 17/02

(54) **Wound protector including pocket for reusable distal ring**

(30) Priority: 23.03.2011 US 201161466567 P; 05.03.2012 US 201213411741
(71) Applicant: Tyco Healthcare Group LP, Mansfield, MA 02048 (US)
(72) Inventor: Smith, Robert C., Middletown, CT Connecticut 06457 (US)
(74) Representative: Soames, Candida Jane

(57) **Abstract**

A surgical device is disclosed, the surgical device including a housing, a tube disposed on the housing, and an elongate member. The housing is adapted for insertion into an incision in tissue and defines a proximal end and a distal end. The tube extends distally from the proximal end of the housing to the distal end of the housing and further circumnavigates the distal end of the housing. The elongate member is insertable into the tube to provide additional rigidity to the tube. The housing has a first state prior to insertion of the elongate member and a second state after insertion of the elongate member. The distal end of the housing is collapsible when in the first state to facilitate insertion into the incision and has additional rigidity when in the second state for securing the housing within the incision.

## Description

### CROSS REFERENCE TO RELATED APPLICATION

The present application claims the benefit of and priority to U.S. Provisional Application Serial No.61/466,567, filed on March 23, 2011, the entire contents of which are incorporated herein by reference.

### BACKGROUND

### Technical Field

The present disclosure relates generally to surgical instruments for use with a seal anchor member. More particularly, the present disclosure relates to an incision or orifice protection apparatus usable with a seal anchor member that provides additional protection for incisions or other natural orifices during minimally invasive surgical procedures and allows for the use of standard sized surgical access portals through incisions of varying size.

### Description of Related Art

Increasingly, many surgical procedures are performed through small incisions in the skin. As compared to the larger incisions typically required in traditional procedures, smaller incisions result in less trauma to the patient. By reducing the trauma to the patient, the time required for recovery is also reduced. Generally, the surgical procedures that are performed through small incisions in the skin are referred to as endoscopic. If the procedure is performed on the patient's abdomen, the procedure is referred to as laparoscopic. Throughout the present disclosure, the term minimally invasive is to be understood as encompassing both endoscopic and laparoscopic procedures.

During a typical minimally invasive procedure, surgical objects, such as surgical access devices (e.g., trocar and cannula assemblies) or endoscopes, are inserted into the patient's body through the incision in tissue. In general, prior to the introduction of the surgical object into the patient's body, insufflation gas is used to enlarge the area surrounding the target surgical site to create a larger, more accessible work area. Accordingly, the maintenance of a substantially fluid-tight seal is desirable so as to inhibit the escape of the insufflation gas and the deflation or collapse of the enlarged surgical site. In response to this, various access devices with sealing features are used during the course of minimally invasive procedures to provide an access for surgical objects to enter the patient's body. Each of these devices is configured for use through a single incision or a naturally occurring orifice (i.e. mouth, anus, or vagina) while allowing multiple instruments to be inserted through the device to access the working space beyond the device.

However, a continuing need exists for protection system to minimize damage to the incision or naturally occurring orifice during a surgical procedure and which also allows for the use of standard sized surgical access portals through incisions or orifices of varying size.

### SUMMARY

A surgical device is disclosed, the surgical device including a housing, a tube disposed on the housing, and an elongate member. The housing is adapted for insertion into an incision in tissue and defines a proximal end and a distal end. The tube extends distally from the proximal end of the housing to the distal end of the housing and further circumnavigates the distal end of the housing. The elongate member is insertable into the tube to provide additional rigidity to the tube. The housing has a first state prior to insertion of the elongate member and a second state after insertion of the elongate member. The distal end of the housing is collapsible when in the first state to facilitate insertion into the incision and has additional rigidity when in the second state for securing the housing within the incision.

The housing may also include a ring circumnavigating the proximal end. The ring is adapted to provide additional rigidity to the proximal end of the housing for securing the housing against an outer surface of tissue. The housing and the ring may also be monolithically formed. The housing may also include a lumen extending longitudinally therethrough and adapted for the reception of surgical objects in a substantially fluid-tight manner. The lumen may also be adapted for the reception of a surgical access portal in a substantially fluid-tight manner. The housing may define a substantially fluid tight seal with the incision in tissue. The tube may alternatively be disposed within the housing and the tube and the housing may also be monolithically formed. The elongate member may also include a tapered portion at a distal end to facilitate insertion into the tube.

A method of use for a surgical device is also disclosed, the method including providing a surgical device including a housing, a tube disposed on the housing, and an elongate member. The housing is adapted for insertion into an incision in tissue and defines a proximal end and a distal end. The tube extends distally from the proximal end of the housing to the distal end of the housing and circumnavigates the distal end of the housing. The elongate member is insertable into the tube to provide additional rigidity to the tube. The housing has a first state prior to insertion of the elongate member and a second state after insertion of the elongate member. The distal end of the housing is collapsible when in the first state to facilitate insertion into the incision and has additional rigidity when in the second state for securing the housing within the incision. The method also includes collapsing the distal end of the housing, inserting the housing into an incision in tissue and inserting the elongate member into the tube, thereby providing additional rigidity to the distal end of the housing and securing the housing in the incision in tissue.

The housing may include a lumen extending longitudinally therethrough, the lumen adapted for the reception of a surgical object or a surgical access portal in a substantially fluid-tight manner and the method may include inserting the surgical object or surgical access portal through the lumen.

Inserting the elongate member into the tube may include inserting the elongate member into the tube until the elongate member at least partially circumnavigates the distal end of the housing. It is also contemplated that the elongate member may fully circumnavigate the distal end of the housing and further that the elongate member may bottom out at the end of the tube. The elongate member may include a tapered portion at a distal end and the step of inserting the elongate member into the tube may include inserting the tapered portion into the tube.

The method may further include removing the elongate member from the tube, thereby allowing the distal end of the housing to be collapsible and may also include removing the housing from the incision.

### BRIEF DESCRIPTION OF THE DRAWINGS

The accompanying drawings, which are incorporated in and constitute a part of this specification, illustrate embodiments of the presently disclosed surgical device, and together with a general description of the presently disclosed surgical device given above, and the detailed description of the embodiments given below, serve to explain the principles of the presently disclosed surgical device.

FIG. 1 is a perspective view of a surgical device in accordance with the present disclosure;

FIG. 2 is a perspective view of the surgical device of FIG. 1 before insertion into an incision in tissue;

FIG. 3 is a perspective view of the surgical device of FIG. 1 during insertion into the incision in tissue;

FIG. 4 is a perspective view of the surgical device of FIG. 1 after insertion into the incision in tissue but before insertion of the elongate member into the tube;

FIG. 5 is a perspective view of the surgical device of FIG. 4 after the elongate member is inserted into the tube;

FIG. 6 is a zoomed in view of a portion of the surgical device of FIG. 5 showing the elongate member within the tube;

FIG. 7 is a side cut-away view of the surgical device of FIG. 4 with a surgical access portal inserted therethrough; and

FIG. 8 is the side cut-away view of the surgical device of FIG. 7 with an alternative surgical access portal inserted therethrough.

### DETAILED DESCRIPTION

Disclosed herein is a surgical device for protecting an incision in tissue or natural orifice during minimally invasive surgery. More specifically a surgical device is disclosed which is insertable into a single incision or orifice in a body and capable of providing a protective layer between the incision or orifice and surgical objects or access portals inserted therethrough. The surgical device also allows standard sized surgical access portals to be used through incisions or natural orifices of varying sizes and specifically allows a surgical access portal to be used through an incision or natural orifice that is larger than the surgical access portal.

Particular embodiments of the presently disclosed surgical device are described in detail with reference to the drawing figures wherein like reference numerals identify similar or identical elements. As used herein, the term "distal" refers to that portion which is farther from the user while the term "proximal" refers to that portion which is closer to the user or surgeon. While the term "incision" is used to describe an opening in tissue through which the disclosed surgical device is inserted it is contemplated that the opening may alternatively be any natural orifice such as, for example, the anus or vagina.

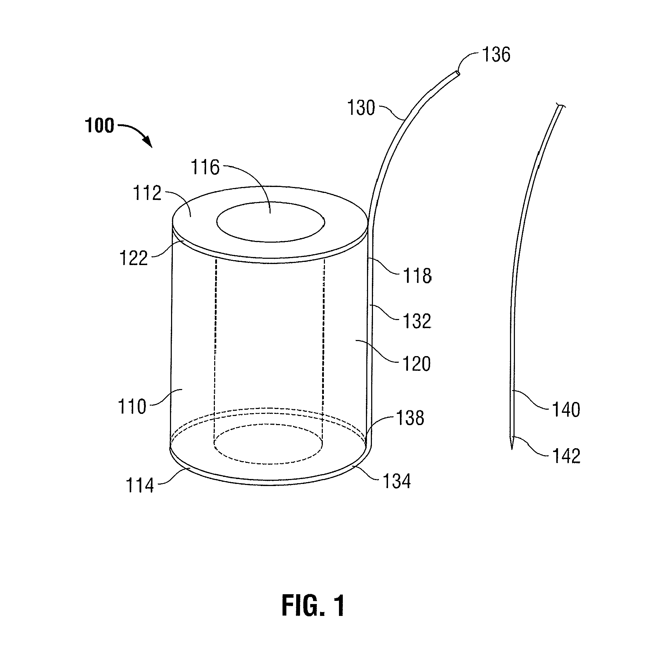

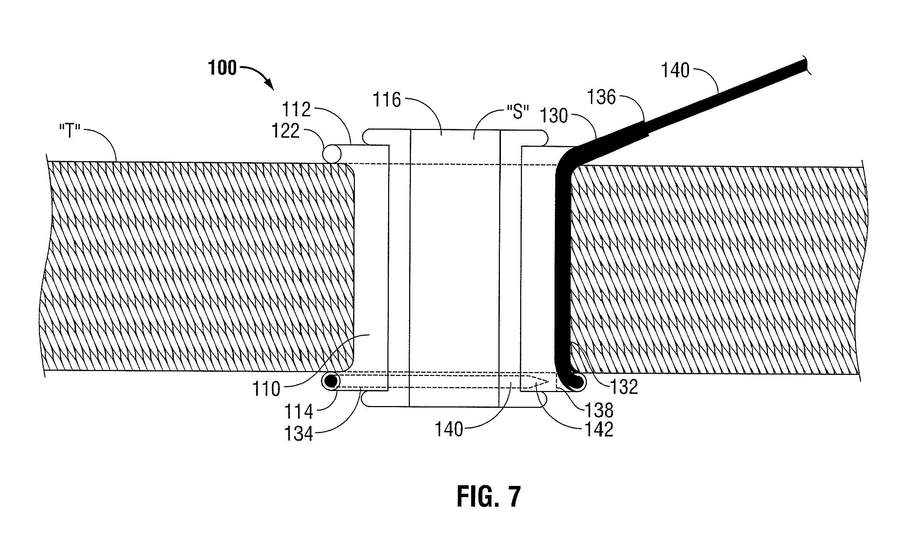

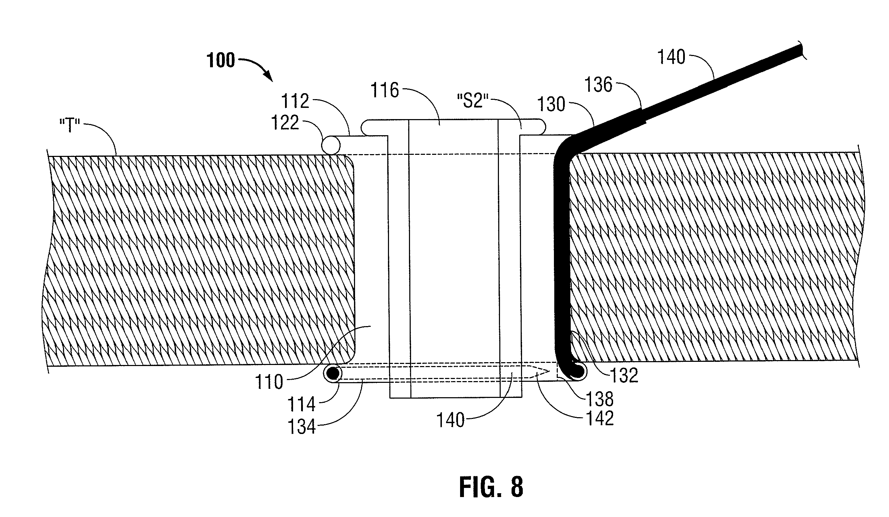

Referring now to FIGS. 1 and 7, a surgical device 100 is disclosed including a housing 110, a tube 130 disposed on housing 110 and an elongate member 140 insertable into tube 130. Housing 110 is adapted for insertion into a natural orifice or an incision in tissue "T" in a substantially fluid-tight manner and defines a proximal end 112 and a distal end 114. Housing 110 may be made of a bio-compatible material which is flexible or collapsible and designed to allow for flexibility or collapsibility during insertion while preventing tearing or damage to the incision or orifice due to movement of instruments or surgical access portals inserted therethrough. It is alternatively contemplated that only a distal portion 120 of housing 110 is made of a flexible or collapsible material with the remaining portion being rigid or semi-rigid. Housing 110 may include a ring 122 circumnavigating proximal end 112 to provide additional rigidity to proximal end 112 and to allow housing 110 to be secured against an outer surface of tissue "T" after insertion. It is contemplated that housing 110 and ring 112 may be monolithically formed. Housing 110 may also define a lumen 116 extending longitudinally therethrough for the fluid-tight reception of surgical objects or a surgical access portal "S" and may include one or more valves (not shown) to seal against the surgical objects, instruments and access portals and/or to prevent the loss of insufflation fluid from the surgical site when the surgical objects, instruments, or surgical access portal "S" are removed. Surgical access portal "S" includes proximal and distal flanged portions and a lumen extending therethrough for the sealed reception of surgical objects or surgical instruments. A suitable access portal "S" is disclosed in U.S. Patent Application No. 12/244,024 which is now incorporated by reference herein. It is also contemplated that a surgical access portal "S2" may instead be used which only has a proximal flange, as can be seen in FIG. 8.

Tube 130 is disposed along an outer surface 118 of housing 110 and defines a longitudinal portion 132 extending from proximal end 112 to distal end 114 and a ring portion 134 extending from the distal end of longitudinal portion 132 and circumnavigating distal end 114 of housing 110. Tube 130 further defines an opening 136 at the proximal end of longitudinal portion 132 for the reception of elongate member 140 and a sealed end 138 disposed at the circumnavigated end of ring portion 134. It is contemplated that tube 130 may instead run through a side lumen (not shown) in housing 110 or that housing 110 and tube 130 may instead be monolithically formed.

Referring now to FIGS. 1 and 5-7, elongate member 140 includes a distal tip 142 and is insertable into tube 130 through opening 136. Elongate member 140 is made of a semi-rigid material and may be inserted through longitudinal portion 132 and into ring portion 134 until it reaches sealed end 138. When inserted, elongate member 140 provides additional rigidity to tube 130 and in particular to distal end 114 of elongate member 140. This allows distal end 114 of housing 110 to be secured against an inner surface of tissue "T". Although elongate member 140 is made of a semi-rigid material it is sufficiently flexible to travel the length of longitudinal portion 132 and ring portion 134 to thereby form a ring like shape at distal end 114 of housing 110. The ability to insert the elongate member 140 *after* the housing has already been positioned within the incision may provide the advantage that the initial insertion of the distal end of the housing 110 through the incision may be more easily accomplished, e.g., because the distal end of the housing 110 has relatively less rigidity and mass than the distal end would have if the distal end had, e.g., an integral distal ring or other structure - while still provide adequate retention of the housing 110 within the incision when the body cavity has been insufflated.

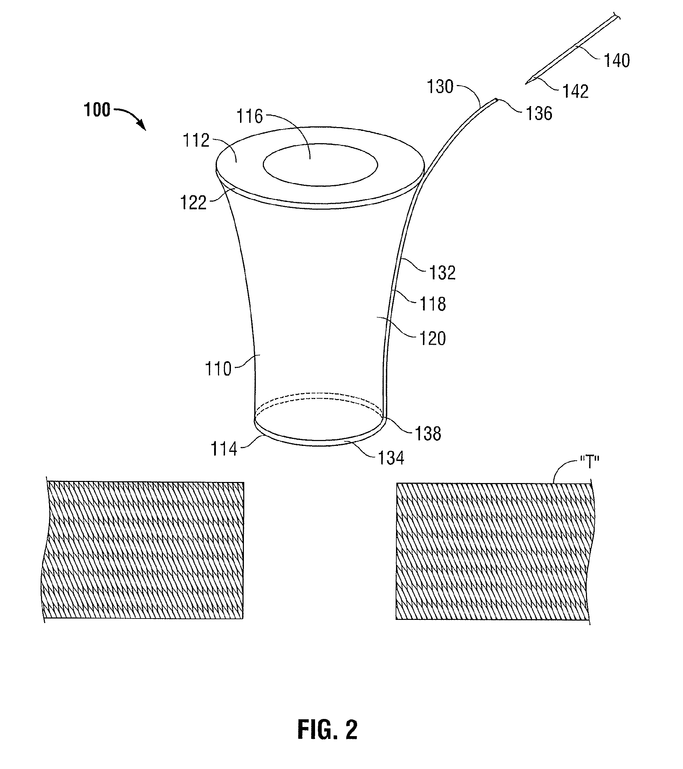

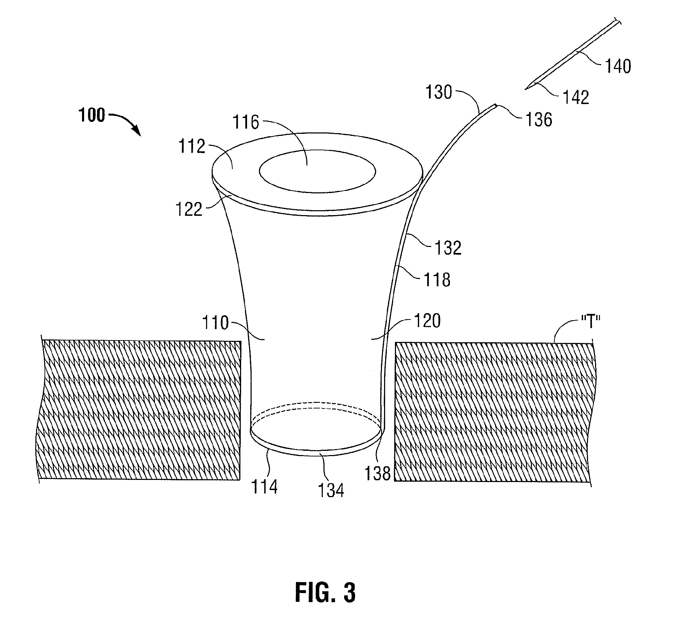

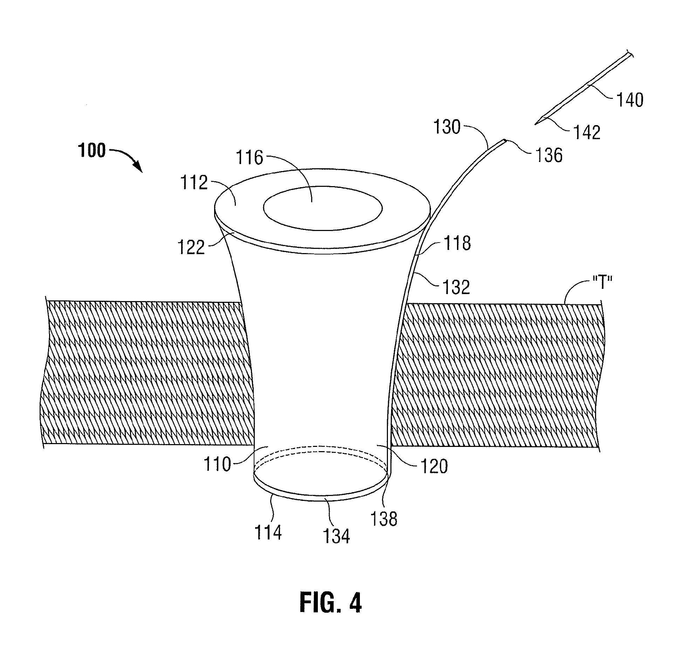

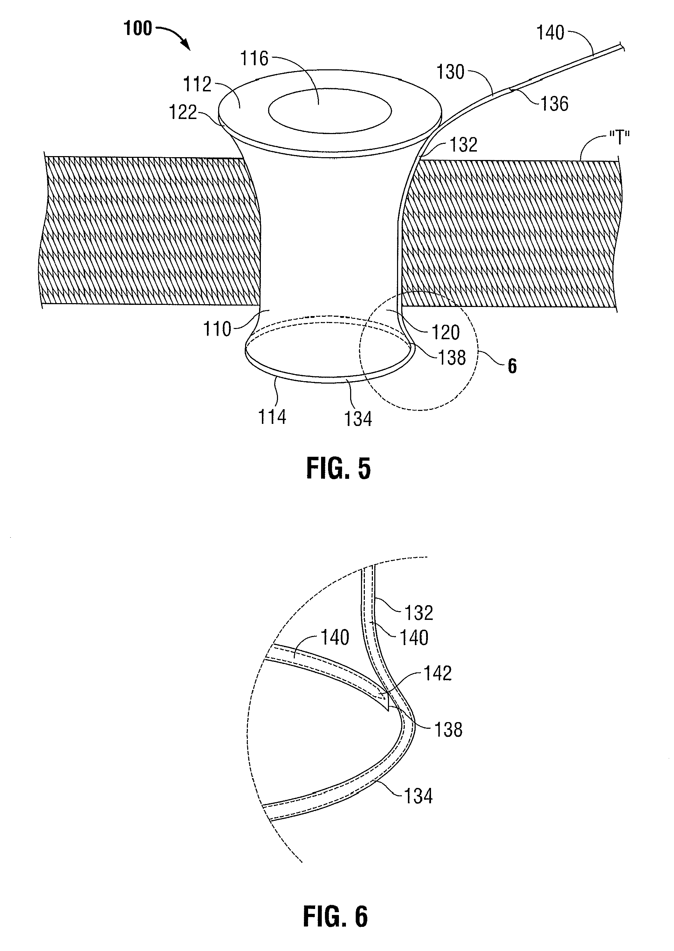

Referring now to FIGS. 1-7, housing 110 and tube 130 have a first state prior to insertion of elongate member 140 (FIG. 2-4), where distal portion 120 of housing 110 is deformable and or flexible and tube 130 is empty, and a second state after insertion of elongate member 140 where distal end 114 of housing 110 is provided with additional rigidity by elongate member 140 filling tube 130 and circumnavigating distal end 114 within tube 130 (FIGS. 5-7). The degree of rigidity in the second state is sufficient to secure housing 110 within the orifice or incision in tissue "T" such that housing 110 may not be withdrawn without first removing elongate member 140 from tube 130. It is further contemplated that a plurality of states are possible where the degree of rigidity of distal end 114 depends on the how far elongate member 140 is inserted into tube 130 and how much of tube 130 remains unfilled. For example, if elongate member 140 is only inserted into tube 130 until it fills longitudinal portion 132, distal end 114 of housing 110 would still remain at least partially collapsible or flexible. Further, if elongate member 140 is inserted into tube 130 such that it only partially fills ring portion 134, the rigidity of distal end 114 of housing 110 would increase but would still be less than the rigidity if elongate member 140 were fully inserted into tube 130 where it fills ring portion 134 and distal tip 142 bottoms out against sealed end 138. In addition it is contemplated that the degree of rigidity of distal end 114 may be controlled by the material of construction of elongate member 140. For example, if a material of construction is of a first rigidity, distal end 114 of housing 110 would be less rigid at each level of insertion as compared to a material of construction of a second higher rigidity.

During use, referring now to FIGS 2-7, with elongate member 140 removed a physician or surgeon collapses distal portion 120 of housing 110 and inserts housing 110 into an orifice or incision in tissue. Once housing 110 has been inserted into the orifice or incision in tissue "T" such that proximal end 112 of housing 110, and ring 122 if included, abuts or is proximate to an outer surface of tissue "T", the physician inserts distal tip 142 of elongate member 140 into proximal opening 136 of tube 130. Elongate member 140 is then inserted through longitudinal portion 132 and into ring portion 134 until distal tip 142 bottoms out against sealed end 138. Elongate member 140 now circumnavigates distal end 114 of housing 110 thereby creating a ring similar to ring 122 for securing distal end 114 of housing 110 against an inner surface of tissue "T". A surgical object or surgical access portal "S" is now inserted through lumen 116 and a surgical operation may be performed. In this way, by using the presently disclosed surgical device, a standard sized surgical access portal may be effectively utilized for a surgical operation even though the surgical access portal is smaller than the incision or orifice. It is contemplated that surgical access portal "S" may be inserted through lumen 116 prior to insertion of housing 110 into the incision, or prior to insertion of elongate member 140 into tube 130. During the surgical operation, the surgeon inserts surgical instruments through the lumen in surgical access portal "S" to access the underlying surgical site. During the surgical operation surgical access portal "S" may be removed by the surgeon to allow for access of larger surgical instruments or objects and further that elongate member 140 may be fully or partially removed from tube 130 to also allow larger surgical instruments or objects to access the surgical space. Both elongate member 140 and surgical access portal "S" may also be re-inserted during the surgical operation. Upon completion of the surgical operation, the surgeon removes surgical access portal "S" from lumen 116 and withdraws elongate member 140 from tube 130, thereby allowing distal portion 120 of housing 110 to once again become collapsible or flexible. Housing 110 may then be removed from the orifice or incision in tissue "T". It is also contemplated that elongate member 140 may be removed from tube 130 prior to removal of surgical access portal "S" from lumen 116 and that housing 110 may be removed from the orifice or incision prior to removing surgical access portal "S" from lumen 116.

Although the present disclosure has been described with respect to preferred embodiments, it will be readily apparent, to those having ordinary skill in the art that changes and modifications may be made thereto without departing from the spirit or scope of the subject apparatus.

The invention may be described by reference to the following numbered paragraphs:-
1. A surgical device comprising:
   a housing adapted for insertion into an incision in tissue, the housing defining a proximal end and a distal end;
   a tube disposed on the housing and extending distally from the proximal end to the distal end, the tube further circumnavigating the distal end of the housing; and
   an elongate member insertable into the tube to provide additional rigidity to the tube, the housing having a first state prior to insertion of the elongate member and a second state after insertion of the elongate member, the distal end of the housing being collapsible when in the first state to facilitate insertion into the incision and having additional rigidity when in the second state for securing the housing within the incision.
2. The surgical device of paragraph 1, wherein the housing further includes a ring circumnavigating the proximal end, the ring adapted to provide additional rigidity to the proximal end of the housing for securing the housing against an outer surface of tissue.
3. The surgical device of paragraph 2, wherein the housing and the ring are monolithically formed.
4. The surgical device of paragraph 1, wherein the housing further includes a lumen extending longitudinally therethrough and adapted for the reception of surgical objects in a substantially fluid-tight manner.
5. The surgical device of paragraph 1, wherein the housing further includes a lumen extending longitudinally therethrough and adapted for the reception of a surgical access portal in a substantially fluid-tight manner.
6. The surgical device of paragraph 1, wherein the tube is disposed within the housing.
7. The surgical device of paragraph 1, wherein the tube and the housing are monolithically formed.
8. The surgical device of paragraph 1, wherein the housing defines a substantially fluid tight seal with the incision in tissue.
9. The surgical device of paragraph 1, wherein the elongate member further includes a tapered portion at a distal end to facilitate insertion into the tube.
10. A method of use for a surgical device, the method comprising the steps of:
   providing a surgical device comprising:
      a housing adapted for insertion into an incision in tissue, the housing defining a proximal end and a distal end;
      a tube disposed on the housing and extending distally from the proximal end to the distal end, the tube further circumnavigating the distal end; and
      an elongate member insertable into the tube to provide additional rigidity to the tube, the housing having a first state prior to insertion of the elongate member and a second state after insertion of the elongate member, the distal end of the housing being collapsible when in the first state to facilitate insertion into the incision and having additional rigidity when in the second state for securing the housing within the incision;
   collapsing the distal end of the housing;
   inserting the housing into an incision in tissue; and
   inserting the elongate member into the tube, thereby providing additional rigidity to the distal end of the housing and securing the housing in the incision in tissue.
11. The method of paragraph 10, wherein the housing further includes a lumen extending longitudinally therethrough, the lumen adapted for the reception of a surgical access portal in a substantially fluid-tight manner.
12. The method of paragraph 11, further including the step of inserting the surgical access portal through the lumen.
13. The method of paragraph 10, wherein the housing further includes a lumen extending longitudinally therethrough, the lumen adapted for the reception of a surgical object in a substantially fluid-tight manner.
14. The method of paragraph 13, further including the step of inserting the surgical object through the lumen.
15. The method of paragraph 10, wherein the step of inserting the elongate member into the tube includes inserting the elongate member into the tube until the elongate member at least partially circumnavigates the distal end of the housing.
16. The method of paragraph 15, wherein the step of inserting the elongate member into the tube includes inserting the elongate member into the tube until the elongate member fully circumnavigates the distal end of the housing.
17. The method of paragraph 16, wherein the step of inserting the elongate member into the tube includes inserting the elongate member into the tube until the elongate member bottoms out at the end of the tube.
18. The method of paragraph 10, wherein the elongate member further includes a tapered portion at a distal end, the step of inserting the elongate member into the tube including inserting the tapered portion into the tube.
19. The method of paragraph 10, further including the step of removing the elongate member from the tube, thereby allowing the distal end of the housing to be collapsible.
20. The method of paragraph 19, further including the step of removing the housing from the incision.

## Claims

1. A surgical device comprising:
a housing adapted for insertion into an incision in tissue, the housing defining a proximal end and a distal end;
a tube disposed on the housing and extending distally from the proximal end to the distal end, the tube further circumnavigating the distal end of the housing; and
an elongate member insertable into the tube to provide additional rigidity to the tube, the housing having a first state prior to insertion of the elongate member and a second state after insertion of the elongate member, the distal end of the housing being collapsible when in the first state to facilitate insertion into the incision and having additional rigidity when in the second state for securing the housing within the incision.

2. The surgical device of claim 1, wherein the housing further includes a ring circumnavigating the proximal end, the ring adapted to provide additional rigidity to the proximal end of the housing for securing the housing against an outer surface of tissue.

3. The surgical device of claim 2, wherein the housing and the ring are monolithically formed.

4. The surgical device of any preceding claim, wherein the housing further includes a lumen extending longitudinally therethrough and adapted for the reception of surgical objects in a substantially fluid-tight manner.

5. The surgical device of any preceding claim, wherein the housing further includes a lumen extending longitudinally therethrough and adapted for the reception of a surgical access portal in a substantially fluid-tight manner.

6. The surgical device of any preceding claim, wherein the tube is disposed within the housing.

7. The surgical device of any preceding claim, wherein the tube and the housing are monolithically formed.

8. The surgical device of any preceding claim, wherein the housing defines a substantially fluid tight seal with the incision in tissue.

9. The surgical device of any preceding claim, wherein the elongate member further includes a tapered portion at a distal end to facilitate insertion into the tube.

10. A method, the method comprising the steps of:
providing a surgical device comprising:
a housing, the housing defining a proximal end and a distal end;
a tube disposed on the housing and extending distally from the proximal end to the distal end, the tube further circumnavigating the distal end; and
an elongate member insertable into the tube to provide additional rigidity to the tube, the housing having a first state prior to insertion of the elongate member and a second state after insertion of the elongate member, the distal end of the housing being collapsible when in the first state and having additional rigidity when in the second state for securing the housing;
collapsing the distal end of the housing; and
inserting the elongate member into the tube, thereby providing additional rigidity to the distal end of the housing and securing the housing in the incision in tissue.

11. The method of claim 10, wherein the housing further includes a lumen extending longitudinally therethrough, the lumen adapted for the reception of a surgical access portal in a substantially fluid-tight manner.

12. The method of claim 11, further including the step of inserting the surgical access portal through the lumen.

13. The method of any of claims 10 to 12, wherein the housing further includes a lumen extending longitudinally therethrough, the lumen adapted for the reception of a surgical object in a substantially fluid-tight manner; preferably further including the step of inserting the surgical object through the lumen.

14. The method of any of claims 10 to 13, wherein the step of inserting the elongate member into the tube includes inserting the elongate member into the tube until the elongate member at least partially circumnavigates the distal end of the housing; preferably wherein the step of inserting the elongate member into the tube includes inserting the elongate member into the tube until the elongate member fully circumnavigates the distal end of the housing.

15. The method of claim 14, wherein the step of inserting the elongate member into the tube includes inserting the elongate member into the tube until the elongate member bottoms out at the end of the tube; and/or wherein the elongate member further includes a tapered portion at a distal end, the step of inserting the elongate member into the tube including inserting the tapered portion into the tube.
